# EUROPÄISCHE PATENTSCHRIFT

(11) **EP 3 287 065 B1**
(45) Veröffentlichungstag und Bekanntmachung des Hinweises auf die Patenterteilung: **02.01.2019**
(21) Anmeldenummer: 16185806.3
(22) Anmeldetag: 26.08.2016
(51) Int. Cl.: A61B 5/00, A61B 7/00, A61H 23/00, A61F 5/56, A61N 1/04, A61N 7/00, A61H 23/02

(54) **GERÄT ZUR ÜBERWACHUNG UND ZUR BEEINFLUSSUNG DES SCHLAFES EINER PERSON**
DEVICE FOR MONITORING AND INFLUENCING THE SLEEP OF A PERSON
APPAREIL DESTINE A SURVEILLER ET A INFLUENCER LE SOMMEIL D'UNE PERSONNE

(43) Veröffentlichungstag der Anmeldung: 28.02.2018
(73) Patentinhaber: Iptasys GmbH, 89081 Ulm (DE)
(72) Erfinder: Kouemou, Guy, 89081 Ulm (DE)
(74) Vertreter: Hentrich Patentanwälte PartG mbB

(56) Entgegenhaltungen:
- WO-A1-2013/091613
- DE-A1-102014 201 694
- US-A1- 2010 318 007
- US-A1- 2015 173 672

## Beschreibung

Die Erfindung betrifft ein Gerät zur Überwachung und zur Beeinflussung des Schlafes einer Person, insbesondere zur Analyse und Therapie von schlafbezogenen Atmungsstörungen bzw. zur Beseitigung des Schnarchens, mit mindestens einem Sensor zur Detektion von die Lage der Person indizierenden Lagesignalen und/oder mit einem Sensor zur Detektion von intrakorporal erzeugten Bioakustiksignalen, mit einem Gurtsystem, das derart ausgelegt ist, den Sensor an oder nahe an der Person anzuordnen, mit einer Kontrolleinheit, die derart ausgelegt ist, die detektierten Signale auszuwerten oder zu verarbeiten, mit mindestens einem durch die Kontrolleinheit aktivierbaren Reizgeber, durch den bei Erkennen einer vorgebbaren Lage der Person und/oder bei Erkennen eines vorgebbaren akustischen Geräuschmusters durch die Kontrolleinheit ein Reiz bei der Person hervorrufbar ist.

Weiterhin wird ein Verfahren zum Betrieb des erfindungsgemäßen Gerätes bzw. zur Überwachung und Beeinflussung des Schlafes einer Person offenbart.

Eine häufig auftretende schlafbezogene Atmungsstörung ist das obstruktive Schlafapnoe-Syndrom (Atemstillstand). Derartige Apnoen treten beispielsweise bei einer starken Entspannung der ringförmigen Muskulatur um die oberen Atemwege auf. Durch den Atemfluss entsteht ein Unterdruck an der Luftröhre, wobei der obere Teil der Luftröhre aufgrund der entspannten Muskulatur nicht mehr in der Lage ist, dem Unterdruck genug Widerstand entgegenzusetzen. Infolgedessen fällt der obere Teil der Luftröhre zusammen, es kommt zu einer Behinderung der Atemwege und der Sauerstoffgehalt des Blutes fällt ab.

Dieses Absinken des Sauerstoffgehalts ist beispielsweise mit einem Gerät messbar, welches bereits aus der DE 10 2008 063 231 A1 bekannt ist. Hierbei sind ein Verfahren und eine Vorrichtung zum Messen von Biosignalen an einem Patienten offenbart, die der Diagnose von schlafbezogenen Atmungsstörungen dienen. Das Gerät kann speziell im Bereich der häuslichen Pflege Anwendung finden, wobei die Sauerstoffsättigung, die Pulsfrequenz, die Rohpulswellen, der Qualitätsindex der Sauerstoffsättigung und optional der nasale Atemfluss und das Schnarchen erfasst werden.

Von Nachteil an diesem Gerät ist die Vielzahl an Messgeräten, die am Patienten angebracht werden müssen, um die vorgenannten Parameter zu erfassen. Denn mit dieser Art der Schlafüberwachung ist immer eine Verkabelung des Patienten verbunden, was dessen Schlafqualität und damit das Messergebnis ungewollt beeinflusst. Es sind dabei eine Atemflussschnarchnasenbrille und ein am Patientenfinger anzuklemmender Pulsoximetriesensor vorgesehen. Dabei ist vor allem die Nasenbrille im Gesicht des Patienten hinderlich, um eine Messung in gewohnter Schlafsituation durchzuführen. Diese Art der Schlafüberwachung liefert zwar gute Ergebnisse zur Diagnose von Schlafkrankheiten, jedoch treten im Verlauf der Langzeitüberwachung immer wieder störende Messfehler auf, die zum Beispiel aus einer Veränderung der Lage des schlafenden Patienten resultieren.

Auch wenn das Gerät für den Heimgebrauch deklariert ist, so ist es immer noch notwendig, die vom Gerät aufgezeichneten Daten in einem Klinikum auszulesen und auswerten zu lassen. Erst anschließend werden von einem Arzt Maßnahmen zur Behandlung der diagnostizierten schlafbezogenen Atmungsstörung getroffen, um den Patienten zu kurieren.

Weiterhin sind aus dem Stand der Technik als Armband gebildete Geräte bekannt, die den Benutzer bei der Detektion von Schnarchgeräuschen mittels Transkutaner Elektrischer Nervenstimulation alarmieren. Das Schnarchen ist aus medizinischer Sicht unkritisch, denn solange der Benutzer schnarcht, ist ein kontinuierlicher Gasfluss zu und von den Atemorganen gewährleistet. Bei einem Atemstillstand ist dieser Gasfluss unterbrochen und auch das Schnarchen setzt aus, wodurch eine Alarmierung des Benutzers ausbleibt. Eine gefährliche Apnoe lässt sich somit mit diesen Geräten nicht feststellen und sie werden daher ausschließlich als Beseitiger des störenden, aber medizinisch unbedenklichen, Schnarchens betrachtet.

Bei dem in der WO 2008/098 365 A1 beschriebenen Gerät wird ein bioakustischer Sensor eingesetzt um das Schnarchen bzw. den Apnoefall durch die Messung der Körpergeräusche innerhalb der Luftröhre zu detektieren. An einem Halsband ist dabei eine Provokationseinheit vorgesehen zur Stimulation der Luftröhre mittels Gleich- bzw. Wechselstrom. Die Bestromung des Patienten soll dabei dem eingetreten Apnoefall und/oder dem Schnarchen entgegenwirken.

Leider ist es nicht möglich das in der vorgenannten Druckschrift gezeigte Gerät bei Patienten mit Herzleiden oder bei Schwangeren einzusetzen, da die der Atmungsstörung entgegenwirkende Reizgebung mittels elektrischer Energie hochgradig gefährlich für diese Risikogruppen ist.

Andererseits ist es jedoch für bestimmte Bevölkerungsgruppen, wie beispielsweise an Adipositas leidende Menschen, nahezu essentiell, eine Reizgebung mittels einer elektrischen Stimulation einzusetzen denn häufig ist diese die einzige Reizgebung auf die sie hinreichend reagieren. Somit kann bei diesen Bevölkerungsgruppen der Atmungsstörung oft nur mittels Elektrostimulation entgegengewirkt werden.

In US 2015/0173672 A1 ist ein Gerät gezeigt und beschrieben, das schlafbezogenen Atmungsstörungen entgegenwirken soll. Hierzu kann ein Körperschallmikrofon vorgesehen sein, welches die biologischen Prozesse wie Herzschlag, Atmung und insbesondere auch Schnarchen detektieren kann. Durch einen akustischen Reizgeber wird der Patient oder Proband über eine schlafbezogene Atmungsstörung informiert, um dieser entgegenzuwirken.

Die DE 10 2014 201 694 A1 beschreibt ein Gerät, welches die Lage einer schlafenden Person erfassen kann. Wenn die Person eine ungesunde Lagesituation einnimmt, so wirkt ein Vibriermittel dieser ungesunden Lagesituation entgegen.

In US 2010/0318007 A1 ist ein Gerät beschrieben, welches in Form eines Gurtes ausgeführt ist. Dieses Gerät erfasst mittels eines Atmungssensors und/oder mittels eines Mikrofons Geräusche einer schlafenden Person. Wenn eine schlafbezogene Atmungsstörung detektiert wird, so wird eine Stimulationseinheit aktiviert, die wiederum Stimulatoren antreibt. Diese Stimulatoren können als Vibratoren ausgeführt sein.

Aus der WO2013/091613A1 ist ein mobiles Überwachungs-, Analyse- und Therapieassistenzgerät bekannt, das sich als sinnvoll erwiesen hat, um im Apnoefall einen der Atmungsstörung entgegenwirkenden Reiz auf den Benutzer auszuüben. Es hat sich herausgestellt, dass der Benutzer bei oder nach der Ausübung dieses Reizes häufig erwacht, was einem erholsamen Schlaf entgegenwirkt.

Der Erfindung liegt somit die Aufgabe zugrunde, ein alternatives Gerät der eingangs genannten Art derart weiterzubilden, welches den Schlaf des Benutzers noch stärker fördert. Weiterhin ist es Aufgabe der vorliegenden Erfindung ein Verfahren zum Betrieb des Geräts bereitzustellen.

Diese Aufgabe wird bei einem Gerät zur Überwachung und zur Beeinflussung des Schlafes einer Person gemäß Patentanspruch 1 gelöst. Der Reizgeber umfasst eine durch die Kontrolleinheit elektrisch steuerbare Trommel mit einer Schwingungsmembran und mit einem der Schwingungsmembran zugeordneten Schlägel, die derart ausgelegt ist, Schallwellen in Form eines Hörschalles auf die Person abzugeben.

Damit macht sich das Gerät zu Nutze, dass Menschen im Schlaf Schallfrequenzen unterschiedlich wahrnehmen. Das Gerät kann individuell an die Bedürfnisse der Person angepasst werden, um einem Apnoefall vorzubeugen und/oder einen solchen zu therapieren. Weiterhin kann das Gerät ein Schnarchen stoppen und/oder therapieren. Zusätzlich kann der gesunde Schlaf durch eine geschickte Frequenzwahl und durch eine geschickte aktive Beschallung durch die Trommel gefördert werden, wobei sich insbesondere Tiefschlafphasen verlängern lassen.

Mit der Erfindung ist weiterhin der Vorteil verbunden, dass die Person bei einer Apnoe durch den Reiz veranlasst ist, ihre Lage zu ändern. Durch diese Lageänderung wird einer zusammengefallenen Luftröhre entgegengewirkt, wodurch die Atemwege wieder frei werden. Außerdem ist es möglich, durch den der Atmungsstörung entgegenwirkenden Reiz eine akustische Stimulation der ringförmigen Muskulatur um die oberen Atemwege zu bewirken, so dass diese nicht erschlaffen und die Atemwege dadurch befreit werden beziehungsweise frei bleiben. Hierbei ist es nicht notwendig, die Person aufzuwecken, denn es genügt in einem solchen Falle meistens, sie aus einer tiefen Schlafphase in eine Höhere zu bewegen, in welcher der Körper über ausreichend, einer Atmungsstörung entgegenwirkenden, Muskelspannung verfügt. Der Sensor zur Detektion von intrakorporal erzeugten Bioakustiksignalen (Bioakustiksensor = Körperschallmikrofon) selbst kann dabei vorzugsweise auf Höhe der Luftröhre angeordnet sein, wo er die Geräusche des Luftflusses zur und von der Lunge detektiert. Der Sensor kann jedoch auch an anderen relevanten, Stellen des Körpers der Person angeordnet werden, an denen Geräusche entstehen, die eine Atmungsstörung und/oder ein Schnarchen akustisch detektierbar machen. Die Kontrolleinheit überprüft kontinuierlich oder in bestimmten Zeitabschnitten und/oder Zeitdauern, ob ein bestimmtes vorgebbares

Geräuschmuster auftritt. Sollte beispielsweise das Geräusch des Gasflusses zur und von der Lunge für eine bestimmte Zeit verstummen, so aktiviert die Kontrolleinheit den Reizgeber. Ebenso verhält es sich, wenn der Sensor zur Detektion von die Lage der Person indizierenden Lagesignalen (Lagesensor) für längere Zeit ein die ungünstige Lage indizierendes Signal an die Kontrolleinheit übermittelt.
Die durch den Sensor detektierten akustischen Signale können in Korrelation mit der Sauerstoffsättigung des Blutes stehen. Daher ist es mit der Erfindung möglich, mittels akustischer Signaldetektion die Sauerstoffsättigung des Blutes festzustellen unter Verzicht auf eine aufwändige, optische Pulsoximetriemessung. Die bioakustische Messung einer Apnoe, die in einer Reduktion des Sauerstoffs und in einer Anreicherung von Kohlenstoffdioxid im Blut resultiert, ist darüber hinaus auch schneller als die optische Pulsoximetriemessung. Durch einen an der Luftröhre angeordneten Bioakustiksensor kann schnell festgestellt werden, ob ein Gasfluss zur und von der Lunge stattfindet. Ist dieser unterbrochen, so sinkt erst nach und nach die Sauerstoffsättigung im Blut ab und es vergeht weitere kostbare Zeit, bis das sauerstoffarme Blut bis zum Finger des Patienten gepumpt wurde, an dem die Apnoe durch einen Pulsoximetriesensor detektiert werden kann.

Sowohl die Schwingungsmembran als auch der Schlägel der Trommel sind mit sehr kleinen Abmessungen gebildet, so dass das Gerät insgesamt kompakt gestaltet ist.

Zur betriebssicheren Verstellung des Schlägels, so dass dieser auf die Schwingungsmembran aufschlagen kann, lässt sich dadurch erreichen, dass die Trommel mittels eines elektrisch verstellbaren Piezoelements oder mittels eines elektromechanischen Kleinmotors betätigbar ist. Auch durch Einsatz dieser Komponenten lässt sich das Gerät mit geringen baulichen Abmessungen bilden, so dass es die Person im Schlaf (nahezu) nicht stört.

Weiterhin ist im Rahmen einer alternativen Ausführungsform vorgesehen, dass der Reizgeber mindestens eine zweite elektrisch betätigbare Trommel umfasst, die ebenfalls derart ausgelegt ist, Schallwellen und/oder Vibrationen auf die Person abzugeben. Damit ist der Vorteil verbunden, dass ein größerer Frequenzbereich für die Trommelschläge der Trommel zur Verfügung gestellt ist, da beispielsweise zwei Trommeln auch einen Wirbel (Trommelwirbel) erzeugen können, so dass dies auf diverse Personengruppen besonders beruhigend einwirkt. Auf andere Personengruppen wirkt dies reizgebend, so dass im Falle einer Apnoe diese hinreichend alarmiert werden.

Zur Realisierung von verschiedenen Schallfrequenzen ist es ebenfalls sinnvoll, wenn die Kontrolleinheit derart ausgelegt ist, eine erste Trommel und die zweite Trommel asynchron zu betätigen.

Es hat sich herausgestellt, dass abgespeicherte Musik- Sequenzen ebenfalls schlaffördernd sind, so dass es sich gemäß einer weiteren Ausführungsform als vorteilhaft erwiesen hat, wenn der Reizgeber ein Lautsprecherelement zur Ausgabe oder Abgabe von zusätzlichen Schallwellen auf die Person umfasst.

Die von der Trommel ausgesendeten Schallfrequenzen sind im Bereich des Infraschall und/oder des Hörschalls und/oder des Ultraschalls, wobei auch Vibrationen auf die Person abgegeben werden können. Dies lässt sich dadurch erzielen, dass die Trommel entsprechend ausgelegt ist.

Eine weitere vorteilhafte Ausführungsform ist dadurch gekennzeichnet, dass der Reizgeber eine elektrische Provokationseinheit umfasst, die vorzugsweise als ein EMS-/TENS-Gerät zur Reizbildung mittels Transkutaner Elektrischer Nervenstimulation und/oder Elektrischer Muskelstimulation gebildet ist. Diese Art der Nerven- beziehungsweise Muskelstimulation ist gut geeignet, um einer Erschlaffung der oberen ringförmigen Atmungsmuskulatur entgegenzuwirken. Selbstverständlich kann das EMS-/TENS-Gerät auch an anderen Stellen des Probandenkörpers, vorzugsweise an den Extremitäten, angeordnet werden. Das EMS-/TENS-Gerät verfügt über Elektroden, die am Probandenkörper angelegt werden können, wobei es vorteilhaft ist, die Elektroden in das Gurtsystem zu integrieren, um das Gerät zur mobilen Überwachung und zur Beeinflussung des Schlafes kompakt zu bilden. Ebenso ist es möglich das EMS-/TENS-Gerät einem Arm- und/oder Beinband zur Befestigung an den Extremitäten zuzuordnen. Die Reizstromtherapie stellt dabei einen besonders geräuscharmen und wirksamen Mechanismus bereit, um den Probanden oder Patienten auf eine Atmungsstörung hinzuweisen. Insbesondere Personen, die mit der Trommel im Extremfall (Apnoenotfall) nicht geweckt beziehungsweise gereizt werden können, reagieren auf die als EMS-/TENS-Gerät gebildete elektrische Provokationseinheit sehr gut.

Im Rahmen der Erfindung ist es weiterhin sinnvoll, wenn beim Erkennen des vorgebbaren akustischen Geräuschmusters oder beim Erkennen der vorgebbaren Lage der Person die Trommel und die Provokationseinheit zeitlich versetzt aktivierbar sind. Somit kann beispielsweise die schonende Trommel zuerst einsetzen, und erst wenn diese schonendere Trommel der Atmungsstörung nicht entgegenwirken kann, wird die elektrische Provokationseinheit hinzugeschaltet.

Es ist von Vorteil, wenn ein Gehäuse und ein dem Gehäuse zugeordneter Wahlschalter vorgesehen sind zur Auswahl eines Betriebsmodus "Akustisch" und/oder "Elektrisch" und/oder "Mechanisch". Damit ist der Vorteil verbunden, dass die Person selbst wählen kann, wie sie das Gerät bertreiben möchte: Ausschließlich mit der Trommel, welche im Hörschallbereich betrieben wird (akustisch), ausschließlich mit der elektrischen Provokationseinheit (elektrisch) oder ausschließlich durch Vibrationseinwirkung auf die Person (mechanisch). Es ist auch möglich, Betriebsmodi vorzusehen, die eine Kombination der vorstehend genannten Betriebsmodi bilden. Günstig ist es, wenn der durch die Trommel hervorgerufene Reiz sukzessive stufenweise oder kontinuierlich verstärkbar ist. So kann die Reizgebung zunächst sehr schonend erfolgen. Sollte die schonende Reizgebung der Atmungsstörung oder dem Schnarchen nicht entgegenwirken können, so wird dies durch das erfindungsgemäße Gerät erkannt, wodurch die Reizgebung verstärkt wird.

Beim Einsatz eines Bioakustiksensors innerhalb eines Gehäuses, hat es sich als zweckmäßig erwiesen, wenn das Gehäuse auf seiner der Person zugewandten Seite eine Transmissionsoberfläche zur Transmission der bioakustischen Signale und auf der der Person abgewandten Seite eine der Schallisolation dienende Isolationsoberfläche aufweist. Hierdurch ist gewährleistet, dass die Umgebungsgeräusche möglichst stark gedämpft werden und vorwiegend oder ausschließlich die Geräusche aus dem Körper der Person durch den Bioakustiksensor detektiert werden. Daher ist es darüber hinaus sinnvoll, wenn der Bioakustiksensor der Transmissionsoberfläche zugeordnet ist, da die Körpergeräusche durch die Lage des Sensors, nach der Art eines Stethoskops eines Arztes, optimal erfasst werden können. Besonders günstig ist es, wenn mindestens ein weiterer Akustiksensor der Isolationsoberfläche zugeordnet ist. Somit kann der der Person zugewandte Bioakustiksensor die bioakustischen Signale des Personenkörpers und der der Person abgewandte Akustiksensor die Umgebungsgeräusche erfassen. Somit ist es möglich Geräusche, die nicht aus dem Personenkörper stammen, eindeutig als Umgebungsgeräusche zu identifizieren. Tritt nämlich ein Umgebungsgeräusch auf, so wird das Geräusch zuerst vom Personenkörper fernen Sensor detektiert, bevor das Geräusch vom Personenkörper nahen Sensor erfasst wird. Der hierdurch messbare Gangunterschied der beiden detektierten Geräuschmuster lässt auf ein Umgebungsgeräusch schließen. Umgekehrt kann auf diese Weise auch ein Körpergeräusch eindeutig als ein solches identifiziert werden, da ein Körpergeräuschsignal am Personenkörper nahen Sensor früher detektiert wird, als am Personenkörper fernen Sensor.
Desweiteren ist es bevorzugt, wenn in der Isolationsoberfläche und/oder in der Transmissionsoberfläche ein Schallkanal ausgebildet ist, denn hierdurch ist die Detektion der Umgebungsgeräusche deutlich erleichtert.

Weiterhin ist es sinnvoll, wenn im Gehäuse eine Schalltrennwand angeordnet ist. So kann ein Personenkörper naher Sensor gut gegen Umgebungsgeräusche und ein Personenkörper ferner Sensor gut gegen Körpergeräusche isoliert werden. Die Schalltrennwand ist vorzugsweise aus einem Schaumstoff und/oder einem Kunststoff gebildet. Es sind auch sonstige bevorzugte aus dem Stand der Technik bekannte Materialien für die Schalltrennwand einsetzbar.

Auch ist es vorteilhaft, wenn zwischen dem Reizgeber und der Kontrolleinheit eine zumindest unidirektionale Kommunikationsverbindung und/oder Ansteuerungsmöglichkeit vorgesehen ist. Hierdurch ist gewährleistet, dass die Trommel durch die Kontrolleinheit sicher und vor allem zuverlässig aktiviert werden können. Die Kommunikationsverbindung kann dabei konventionell drahtgebunden oder drahtlos mittels Bluetooth, Wireless-LAN, Funk oder dergleichen gebildet sein.

Um die Kontrolleinheit besonders kompakt und klein zu bilden, hat es sich als besonders sinnvoll erwiesen, die Kontrolleinheit KI-fähiger (KI=künstliche Intelligenz) Technologieprozessor zu bilden auf dem eine eine Deeplearning Methode anwendende Software ausgeführt wird. Insbesondere ist die Kontrolleinheit als ein Mikrocontroller oder ASIC (anwendungs-spezifisch integrierte Schaltung) oder FPGA (feldprogrammierbare Gatter-Anordnung) oder FPAA (feldprogrammierbare Analoganordnung) gebildet.

Weiterhin ist sinnvoll, wenn mindestens eine dem Speichern und/oder Auslesen von Daten dienende Speichereinheit vorgesehen ist. Hierdurch ist es möglich, über Nacht Daten aufzuzeichnen, um diese am nächsten Tag beispielsweise mittels eines externen PCs auszuwerten. Auch sind vorzugsweise elektronische Schnittstellen am Gerät vorgesehen, die ein Auslesen der Daten ermöglichen bzw. eine Telemedizin bereit stellen. Diese können als USB- oder LAN-Anschlüsse oder dergleichen gebildet sein, wobei auch die Verwendung einer drahtlosen Verbindung wie Bluetooth, W-LAN oder dergleichen möglich ist. Weiterhin ist es möglich das Messergebnis an ein Smartphone zu übertragen, das in einer Applikation (App) die Messdaten als aufbereitetes Ergebnis an die Person ausgibt. Auch kann die Speichereinheit als Wechseldatenträger beispielsweise als Flash-EEPROM (Flash-electrically erasable programmable read-only memory) ausgeführt sein, vorzugsweise als Secure Digital Memory Karte (SD-Karte), als Compact Flash-Karte, als SmartMedia-Karte (SM-Karte) oder dergleichen. In einer derartigen Ausführungsform ist ein Steckplatz für die entsprechende Karte am Mikrocontroller vorgesehen und das Gehäuse verfügt über eine entsprechende Kartenaufnahme.

Von Vorteil ist es außerdem, wenn der Mikrocontroller mindestens ein Filtersystem zur Geräuschartefaktefilterung zugeordnet ist. Hierdurch können Umgebungsgeräusche im Raum, die den Atemfluss überdeckenden Körpergeräusche, wie beispielsweise der Herzschlag und auch die Geräusche, die aus der Bewegung der Person bei der Respiration und der Inspiration entstehen, herausgefiltert werden.

Bevorzugt ist das Filtersystem als ein ein- oder mehrkanalig, hard- und/oder softwarebasierter Filter gebildet. Die Geräuschartefaktefilterung kann dabei über eine entsprechend konfigurierte feldprogrammierte Analoganordnung (FPAA) geschehen. Zur Artefaktebeseitigung werden verschiedene CAB ("configurable analog blogs") der FPAA in einer ein- oder mehrdimensionalen Matrix positioniert. Diese CABs weisen einen Eingang und einen Ausgang, sowie mindestens einen Operationsverstärker, konfigurierbare Kapazitäten und Widerstände, konfigurierbare Verbindungsresourcen (Leiter, Schaltungen) und einen Konfigurationsdatenspeicher auf. Jeder CAB der FPAA ist als ein Bandpass oder als eine Bandsperre konfiguriert, um bestimmte Frequenzbereiche des Eingangssignals herauszufiltern, die zuvor als artefakte Frequenzbereiche definiert wurden. Beispielsweise werden die FPAA-Eingangs-Geräusche, die der Patient bei der Bewegung im Schlaf generiert, durch einen der CABs herausgefiltert, wodurch das Ausgangssignal der FPAA von Bewegungsartefakten befreit ist.

Weiterhin ist vorteilhaft, wenn ein mit dem KI-Technologieprozessor (mit z.B. Markov-Prozess), dem Mikrocontroller oder ASIC oder FPGA oder FPAA verbundener interner oder externer Analog-Digital-Wandler vorgesehen ist. Somit kann die Auswertung der Daten auch bereits im Gerät zur mobilen Schlafüberwachung durchgeführt werden. Der Bioakustiksensor kann dabei integral mit dem Analog-Digital-Wandler gebildet sein. Dies erhöht darüber hinaus die Kompatibilität des erfindungsgemäßen Geräts mit einer Vielzahl an unterschiedlichen Mikrocontrollern. Der Mikrocontroller seinerseits kann auch integral mit einer feldprogrammierbaren Gatter-Anordnung (FPGA) gebildet sein, wobei dieser digitale integrierte Schaltkreis derart konfiguriert werden kann, dass er auf Miniaturbasis aufwändige Rechenoperationen auf Grundlage eines lernfähigen, einspielbaren und/oder ersetzbaren Algorithmus durchführt.

Weiterhin ist bevorzugt, wenn Mittel zur Kalibrierung vorgesehen sind, die vorzugsweise automatisch betreibbar sind. Hierdurch können im Vorhinein Umgebungsgeräusche aufgezeichnet werden, die bei der Messung an der Person durch das Filtersystem herausgefiltert werden können.

Weiterhin ist vorteilhaft, wenn mindestens ein Bedienelement vorgesehen ist, welches vorzugsweise am Gehäuse angeordnet ist. Dieses Bedienelement kann dem Ein- bzw. Ausschalten des Gerätes dienen, wobei desweiteren eine Kalibrierung durch beispielsweise längeres Drücken des Gerätes erfolgt.

Eine bevorzugte Ausführungsform ist dadurch gekennzeichnet, dass das Gurtsystem aus textilem Material gebildet ist und Schnallen zum Verschließen und/oder zur Längeneinstellung der Gurte des Gurtsystems umfasst. Damit ist sichergestellt, dass die Sensoren optimal am Körper des Benutzers anliegen. Textile Materialien fühlen sich auf der Haut angenehmer an, als beispielsweise Kunststoffmaterialen, so dass die Mittel zur Lagefixierung für den Benutzer angenehm zu tragen sind. Auch sind textile Materialien optimal, wenn Schweißbildung, insbesondere bei langem Tragen Verschließen und/oder zur Längeneinstellung der Gurte des Gurtsystems umfasst. Damit ist sichergestellt, dass die Sensoren optimal am Körper des Benutzers anliegen. Textile Materialien fühlen sich auf der Haut angenehmer an, als beispielsweise Kunststoffmaterialen, so dass die Mittel zur Lagefixierung für den Benutzer angenehm zu tragen sind. Auch sind textile Materialien optimal, wenn Schweißbildung, insbesondere bei langem Tragen auftritt. Auch Biozide, Pilze und Bakterien können bei textilen Brustgurten nicht so schnell entstehen. Besonders günstig ist es dabei, wenn das Gurtsystem waschbar ist. Diese Reinigungsmöglichkeit bietet hygienische Vorteile für den Probanden. Bei den Mitteln zur Lagefixierung ist es besonders sinnvoll, wenn diese längenverstellbar gebildet sind und der Festlegung der Länge dienende Schließen, beispielsweise in Form von Schnallen oder anderen Befestigungsmittel aufweisen.

Im Folgenden wird die Erfindung an in der Zeichnung dargestellten Ausführungsbeispielen näher erläutert; es zeigen:

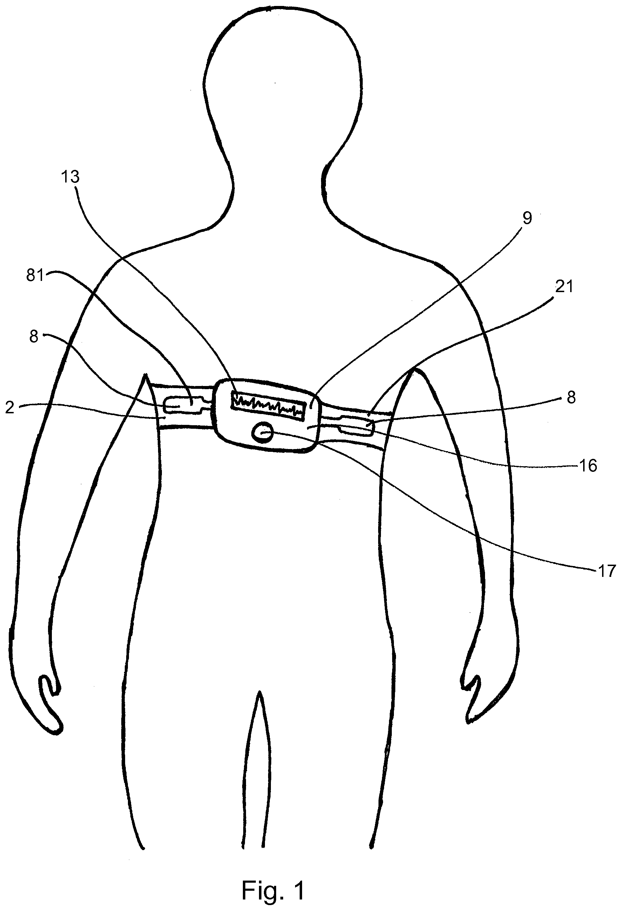

- Fig. 1: ein erstes Ausführungsbeispiel des erfindungsgemäßen Gerätes angeordnet an einer Person in einer Frontansicht,
- Fig. 2: das Gerät aus Figur 1 in einer Seitenansicht, teils geschnitten gezeigt und
angeordnet ist. Weiterhin ist ein Gurtsystem 2 vorgesehen, das derart ausgelegt ist, den Bioakustiksensor 1 sehr nahe an der Person anzuordnen. Über eine Leitung ist der Bioakustiksensor 1 mit einer Kontrolleinheit 3 verbunden und die Kontrolleinheit 3 ist derart ausgelegt, die vom Bioakustiksensor 1 detektierten Signale auszuwerten und zu verarbeiten. Weiterhin ist ein Reizgeber 4 vorhanden, durch den bei Erkennen eines vorgebaren akustischen Geräuschmusters durch die Kontrolleinheit 3 ein Reiz bei der Person hervorrufbar ist. Erfindungsgemäß weist der Reizgeber 4 eine durch die Kontrolleinheit 3 elektrisch steuerbare Trommel 5 auf, die derart ausgelegt ist, Schallwellen und/oder Vibrationen auf die Person abzugeben.

Vorliegend weist die Trommel 5 mindestens eine Schwingungsmembran 51 und mindestens einen der Schwingungsmembran 51 zugeordneten Schlägel 52 auf. Der Schlägel 52 der Trommel 5 ist mit einem elektromechanischen Kleinmotor 6 betätigbar derart, dass der Schlägel 52 auf die Schwingungsmembran 51 in vorgebaren Rhythmus bzw. in vorgebaren Frequenzen aufschlägt. Wird durch die Kontrolleinheit 3 ein vorgebaren akustischen Geräuschmuster erkannt, so wird der elektromechanische Kleinmotor 6 betrieben und der Schlägel 52 klopft auf die Schwingungsmembran 51. Dieses Klopfen mit unterschiedlich vorgebaren Schallfrequenzen dient der Schlafförderung.

Vorliegend ist die Spannungsversorgung als ein Akkumulator gebildet, kann aber auch als eine einfache und austauschbare Batterie oder als eine andere aus der Stand der Technik bekannte elektrische Speicherzelle gebildet sein. Von der Kontrolleinheit 3 zu der Trommel 5 des Reizgebers 4 ist eine unidirektionale Kommunikationsverbindung wirksam. Weiterhin ist im gezeigten Ausführungsbeispiel eine elektrische Provokationseinheit 8 vorgesehen, welche als ein EMS-/TENS-Gerät zur Reizbildung mittels Transkutaner elektrische Nervenstimulation und/oder elektrischer Muskelstimulation gebildet ist. Weiterhin ist zwischen der Kontrolleinheit 3 und der elektrischen Provokationseinheit 8 eine unidirektionale Kommunikationsverbindung wirksam. Die Elektroden 81 des EMS-/TENS-Gerätes sind in das Gurtsystem 2 integriert, während die Trommel 5 im Gehäuse 9 angeordnet ist. Alle gezeigten Ausführungsbeispiele besitzen jeweils eine Anzeigeeinheit 13, auf der die momentan gemessenen akustischen Parameter, der Energieladestatus der im Gehäuse 9 angeordneten elektrischen Speicherzelle, der Mess-Modus oder andere für den Fachmann in bekannter Weise anzuzeigende Werte dargestellt werden.

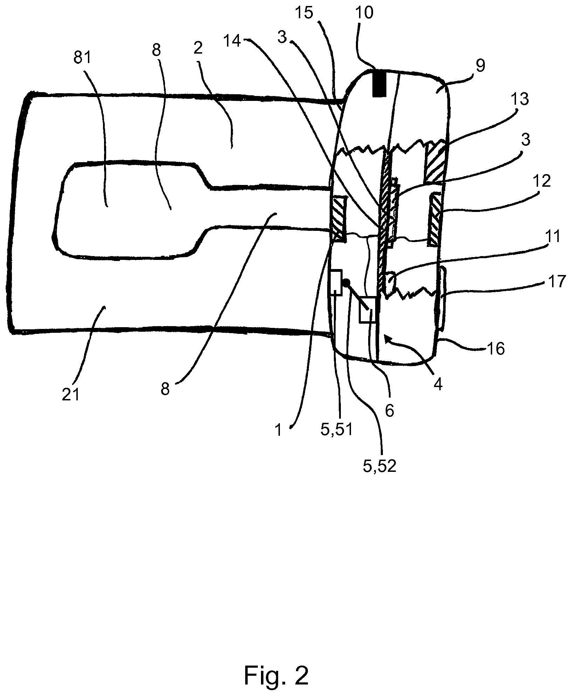

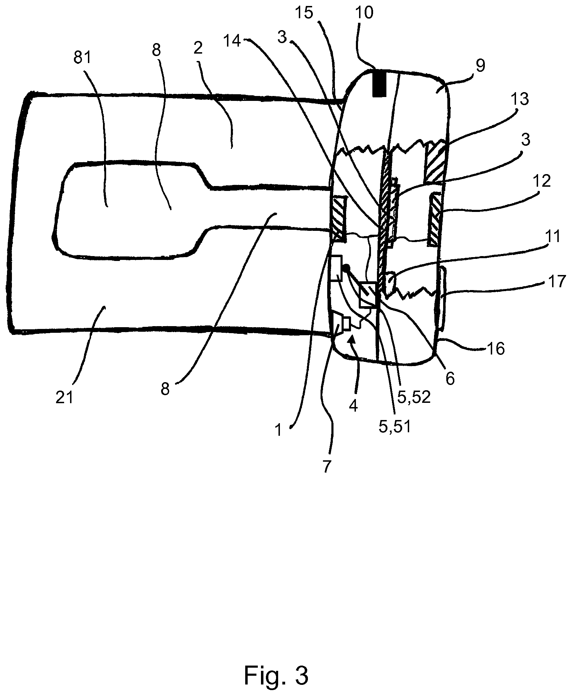

Wie aus den Figuren 2 und 3 ersichtlich wird, ist das gezeigte Gehäuse 9 auf seiner der Person zugewandten Seite mit einer Transmissionsoberfläche 15 zur Transmission der bioakustischen Signale aus dem Personenkörper gebildet. Im gezeigten Ausführungsbeispiel ist innerhalb des Gehäuses 9 eine Schalltrennwand 14 angeordnet, die die Umgebungsgeräusche, das heißt die Geräusche von außen und die Körpergeräusche der Person von innen, absorbiert. Hierbei ist also der Bioakustiksensor 1 vorgesehen zur Detektion intrakorporal erzeugten Schalls und der Akustiksensor 12 dient der Detektion von Umgebungsgeräuschen.

Die Kontrolleinheit 3 ist als ein KI-fähiger Technologieprozessor (Mikrocontroller) gebildet und das Gerät weist desweiteren eine dem Speichern und Auslesen von Daten dienende Speichereinheit 11 auf. Auf dem Mikrokontroller sind stochastisch adaptive Filter vorgesehen, die Geräusch-Artefakte herausfiltern. An dem Gehäuse 9 ist ein Einknopf Bedienelement 17 ausgebildet, welches gedrückt und/oder gedreht werden kann, um das Gerät zwischen den Betriebsmodi umzuschalten oder dieses ein- und auszuschalten. Die Ausführungsform des Gerätes nach Figur 3 unterscheidet sich von demjenigen nach Figur 2 dadurch, dass der Reizgeber 4 zusätzlich ein Lautsprecherelement 7 umfasst zur Abgabe von zusätzlichen Schallwellen auf die Person. In der Speichereinheit 11 können Musik- Sequenzen abgelegt sein, die bei deren Abspielen den Schlaf der Person fördert.

Nachfolgend soll die Betriebsweise des Gerätes zur Überwachung und zur Beeinflussung des Schlafes der Person näher erläutert werden.

Der Bioakustiksensor 1 detektiert kontinuierlich intrakorporal (das heißt innerhalb des Körpers) erzeugte Geräusche und wandelt sie in Bioakustiksignale um. Der Akustiksensor 12 detektiert kontinuierlich Geräusche der Umgebung, so dass Umgebungsgeräusche in Umgebungsgeräuschsignale gewandelt werden. Beide Signale werden der Kontrolleinheit 3 zugeführt. In der Speichereinheit 11 ist eine Datenbank hinterlegt, die die meisten Geräuschartefakte vorgespeichert hat. Diese können aber auch durch eine vorherige Kalibrierung in der Speichereinheit 11 hinterlegt werden.

Der körpernah angeordnete Sensor 1 detektiert im gezeigten Ausführungsbeispiel vorwiegend die intrakorporal entstehenden Geräusche der Luftröhre. Wenn eine Apnoe auftritt, so wird vom inneren Sensor 1 ein vorbekanntes und in der Speichereinheit 11 gespeichertes Geräuschmuster, z. B. eine bestimmte Ruhepause detektiert. Dieses Geräuschmuster wird von der Kontrolleinheit 3 erkannt, woraufhin er über die unidirektionale Kommunikationsverbindung zunächst die Trommel 5 aktiviert. Wird die Atmungsstörung nicht innerhalb eines vorgegebenen Zeitraums beseitigt, so wird anschließend über die unidirektionale Kommunikationsverbindung das EMS-/TENS-Gerät aktiviert, wodurch die Person geweckt wird.

Alternativ lässt sich das Gerät wie folgt einsetzen: Bei Überwachung des Luftflusses der Luftröhre kann festgestellt werden, ob dieser Luftfluss besonders schnell und unruhig erfolgt und/oder ob regelmäßige, insbesondere schnelle Herzgeräusche durch den Bioakustiksensor detektierbar sind. In diesem Fall wird eine vorgegebene Schallfrequenz durch die Trommel 5 auf den Personenkörper abgegeben, um die Person zu beruhigen und insbesondere durch die aktive Beschallung wieder in die Tiefschlafphase zu bringen. Sollte die Trommel 5 nicht ausreichen, so lässt sich das Lautsprecherelement 7 zuschalten, das zusätzlich beruhigende Musik - Sequenzen wiedergibt.
Im Rahmen der Erfindung ist es sinnvoll, wenn die Frequenzen der Trommeleinwirkungen variierbar sind, wodurch das Gerät bei verschiedenen Personen einsetzbar ist, da Menschen unterschiedliche Schallfrequenzen unterschiedlich wahrnehmen.

### Bezugszeichenliste:

- 1: Sensor (=Bioakustiksensor)
- 2: Gurtsystem
- 21: Gurt
- 3: Kontrolleinheit
- 4: Reizgeber
- 5: Trommel
- 51: Schwingungsmembran
- 52: Schlägel
- 6: Kleinmotor
- 7: Lautsprecherelement
- 8: elektrische Provokationseinheit
- 81: Elektrode
- 9: Gehäuse
- 10: Schnittstelle
- 11: Speichereinheit
- 12: Akustiksensor (Sensor)
- 13: Anzeigeeinheit
- 14: Schalltrennwand
- 15: Transmissionsoberfläche
- 16: Isolationsoberfläche
- 17: Bedienelement

## Patentansprüche

1. Gerät zur Überwachung und zur Beeinflussung des Schlafes einer Person, mit mindestens einem Sensor zur Detektion von die Lage der Person indizierenden Lagesignalen und/oder mit einem Sensor zur Detektion von intrakorporal erzeugten Bioakustiksignalen, mit einem Gurtsystem (2), das derart ausgelegt ist, den Sensor (1) an oder nahe an der Person anzuordnen, mit einer Kontrolleinheit (3), die derart ausgelegt ist, die detektierten Signale auszuwerten oder zu verarbeiten, mit mindestens einem durch die Kontrolleinheit (3) aktivierbaren Reizgeber (4), durch den bei Erkennen einer vorgebbaren Lage der Person und/oder bei Erkennen eines vorgebbaren akustischen Geräuschmusters durch die Kontrolleinheit (3) ein Reiz bei der Person hervorrufbar ist, **dadurch gekennzeichnet, dass** der Reizgeber (4) eine durch die Kontrolleinheit (3) elektrisch steuerbare Trommel (5) mit einer Schwingungsmembran (51) und mit einem der Schwingungsmembran (51) zugeordneten Schlägel (52) umfasst, die derart ausgelegt ist, Schallwellen in Form eines Hörschalls auf die Person abzugeben.

2. Gerät nach Anspruch 1, **dadurch gekennzeichnet, dass** die Trommel (5) mittels eines elektrisch verstellbaren Piezoelements oder mittels eines elektromechanischen Kleinmotors (6) betätigbar ist.

3. Gerät nach Anspruch 1 oder 2, **dadurch gekennzeichnet, dass** der Reizgeber (4) mindestens eine zweite elektrisch betätigbare Trommel umfasst, die derart ausgelegt ist, Schallwellen und/oder Vibrationen auf die Person abzugeben.

4. Gerät nach Anspruch 3, **dadurch gekennzeichnet, dass** die Kontrolleinheit (3) derart ausgelegt ist, eine erste Trommel und die zweite Trommel asynchron zu betätigen.

5. Gerät nach einem der Ansprüche 1 bis 4, **dadurch gekennzeichnet, dass** der Reizgeber (4) ein Lautsprecherelement (7) zur Abgabe von zusätzlichen Schallwellen auf die Person umfasst.

6. Gerät nach einem der Ansprüche 1 bis 5, **dadurch gekennzeichnet, dass** der Reizgeber (3) mindestens eine elektrische Provokationseinheit (8) umfasst, welche vorzugsweise als ein EMS-/TENS-Gerät (11) zur Reizbildung mittels Transkutaner Elektrischer Nervenstimulation und/oder Elektrischer Muskelstimulation gebildet ist.

7. Gerät nach einem der Ansprüche 1 bis 6, **dadurch gekennzeichnet, dass** ein Gehäuse (1) und ein dem Gehäuse (1) zugeordneter Wahlschalter vorgesehen sind zur Auswahl eines Betriebsmodus "Akustisch" und/oder "Elektrisch" und/oder "Mechanisch".

8. Gerät nach einem der Ansprüche 1 bis 7, **dadurch gekennzeichnet, dass** der durch die Trommel (5) hervorgerufene Reiz, sukzessive stufenweise oder kontinuierlich verstärkbar ist.

9. Gerät nach einem der Ansprüche 1 bis 8, **dadurch gekennzeichnet, dass** mindestens eine elektronische Schnittstelle (10) vorgesehen ist.

10. Gerät nach einem der Ansprüche 1 bis 9, **dadurch gekennzeichnet, dass** die Kontrolleinheit (3) als ein KI-fähiger Technologieprozessor gebildet ist.

## Claims

1. Device for monitoring and influencing the sleep of a person, with at least one sensor for the detection of position signals indicating the position of the person and/or with a sensor for the detection of intracorporally-generated bioacoustic signals, with a belt system (2), which is configured in such a way as to arrange the sensor (1) at or close to the person with a monitor unit (3) which is configured in such a way as to assess or to process the detected signal, with at least one stimulus generator (4) which can be activated by the monitor unit (3), by means of which, on recognition of a predeterminable position of the person and/or on recognition of a predeterminable sound pattern by the monitor unit (3), a stimulus can be induced for the person, **characterised in that** the stimulus generator (4) comprises a drum (5) which is electrically controllable by the monitor unit (3) with an oscillating diaphragm (51) and with a hammer (52) allocated to the oscillating diaphragm (51), which is configured in such a way that sound waves in form of an audible sound are emitted onto the person.

2. Device according to claim 1, **characterised in that** the drum (5) can be actuated by means of an electrically adjustable piezo element or by means of a small electromechanical motor (6).

3. Device according to claim 1 or 2, **characterised in that** the stimulus generator (4) comprises at least one second electrically actuatable drum, which is configured in such a way as to emit sound waves and/or vibrations onto the person.

4. Device according to claim 3, **characterised in that** the monitor unit (3) is configured in such a way as to actuate a first drum and the second drum asynchronously.

5. Device according to any ones of claims 1 to 4, **characterised in that** the stimulus generator (4) comprises a loudspeaker element (7) for emitting additional sound waves onto the person.

6. Device according to any ones of claims 1 to 5, **characterised in that** the stimulus generator (3) comprises at least one electrical provocation unit (8), which is preferably configured as an EMS/TENS device (11) for creating stimulus by means of transcutaneous electrical nerve stimulation and/or electrical muscle stimulation.

7. Device according to any ones of claims 1 to 6, **characterised in that** a housing (1) and a selector switch allocated to the housing (1) are provided, in order to select an operating mode of "acoustic" and/or "electrical" and/or "mechanical".

8. Device according to any ones of claims 1 to 7, **characterised in that** the stimulus induced by the drum (5) can be amplified successively, in stages, or continuously.

9. Device according to any ones of claims 1 to 8, **characterised in that** at least one electronic interface (10) is provided.

10. Device according to any ones of claims 1 to 9, **characterised in that** the monitor unit (3) is configured as a KI-capable technology processor.

## Revendications

1. Appareil destiné à surveiller et à influencer le sommeil d'une personne, comprenant au moins un capteur destiné à détecter des signaux de position indiquant la position de la personne, et/ou comprenant un capteur destiné à détecter des signaux bioacoustiques générés de façon intracorporelle, comprenant un système de sangle (2) qui est conçu pour disposer le capteur (1) sur la personne ou à proximité de celle-ci, comprenant une unité de contrôle (3) qui est conçue pour évaluer ou traiter les signaux détectés, comprenant au moins un stimulateur (4) qui peut être activé par l'unité de contrôle (3) et qui permet de provoquer une stimulation sur la personne en cas d'identification d'une position de la personne pouvant être prédéterminée et/ou en cas d'identification d'un modèle de bruit acoustique pouvant être prédéterminé, **caractérisé en ce que** le stimulateur (4) comporte un tambour (5) qui peut être commandé par voie électrique par l'unité de contrôle (3) et comprend une membrane vibrante (51) et une baguette (52) associée à la membrane d'oscillation (51) et qui est conçu pour transmettre des ondes sonores à la personne, sous la forme d'un son audible.

2. Appareil selon la revendication 1, **caractérisé en ce que** le tambour (5) peut être actionné au moyen d'un élément piézoélectrique réglable par voie électrique ou au moyen d'un petit moteur (6) électromécanique.

3. Appareil selon la revendication 1 ou 2, **caractérisé en ce que** le stimulateur (4) comprend au moins un deuxième tambour à actionnement électrique qui est conçu pour transmettre des ondes sonores et/ou des vibrations à la personne.

4. Appareil selon la revendication 3, **caractérisé en ce que** l'unité de contrôle (3) est conçue pour actionner de façon asynchrone un premier tambour et le deuxième tambour.

5. Appareil selon une des revendications 1 à 4, **caractérisé en ce que** le stimulateur (4) comprend un élément haut-parleur (7) destiné à transmettre des ondes sonores supplémentaires à la personne.

6. Appareil selon une des revendications 1 à 5, **caractérisé en ce que** le stimulateur (3) comprend au moins une unité de provocation (8) qui est réalisée de préférence comme appareil EMS/TENS (11) pour la génération de stimuli à l'aide de la neurostimulation électrique transcutanée et/ou de l'électrostimulation musculaire.

7. Appareil selon une des revendications 1 à 6, **caractérisé en ce qu'**il est prévu un boîtier (1) et un commutateur de sélection associé au boîtier (1), en vue de sélectionner un mode de fonctionnement « acoustique » et/ou « électrique » et/ou « mécanique ».

8. Appareil selon une des revendications 1 à 7, **caractérisé en ce que** la stimulation provoquée par le tambour (5) peut être amplifiée successivement par paliers ou de manière continue.

9. Appareil selon une des revendications 1 à 8, **caractérisé en ce qu'il** est prévu au moins une interface (10) électronique.

10. Appareil selon une des revendications 1 à 9, **caractérisé en ce que** l'unité de contrôle (3) est réalisée sous forme de processeur technologique adapté à l'IA.
